# EUROPEAN PATENT APPLICATION

(11) **EP 4 589 604 A1**
(43) Date of publication of application: **23.07.2025**
(21) Application number: 23871334.1
(22) Date of filing: 23.06.2023
(51) Int. Cl.: G16H 80/00

(54) **TREATMENT APP MANAGEMENT SYSTEM AND TREATMENT APPLICATION PROGRAM**

(30) Priority: 30.09.2022 JP 2022158381
(71) Applicant: Susmed, Inc., Tokyo 103-0023 (JP)
(72) Inventor: MOTOHASHI, Tomomitsu, Tokyo 103-0023 (JP); TAKAJO, Kentaro, Tokyo 103-0023 (JP)
(74) Representative: Cabinet Chaillot
(86) International application number: PCT/JP2023/023274
(87) International publication number: WO 2024/070089

(57) **Abstract**

A treatment application 1 installed in a patient terminal 10, and a management server 30 that issues an ID are included. The management server 30 includes a medical institution ID issuing unit 31 that issues a medical institution ID for identifying a medical institution that prescribes the treatment application 1, and the treatment application 1 includes an application setting unit 12 that sets a medical institution ID as information for making the treatment application 1 available for use. By using the medical institution ID that does not include information on a patient as an ID required for initiation of use of the treatment application 1, a medical professional in a medical institution that diagnoses the patient and prescribes the treatment application 1 does not perform any input work for issuing the ID, and the medical institution ID can be issued by the management server 30.

## Description

### Technical Field

The present invention relates to a treatment application management system and a treatment application program, and is particularly suitable for use in a system that performs a process relating to initiation of use of a treatment application that is installed and used in a patient terminal.

### Background Art

In recent years, development of an application program aimed at treatment of an illness (hereinafter, referred to as "treatment application") is in progress. For example, a treatment application for treatment of an illness such as sleep disorder through an improvement of a patient lifestyle is known (for example, refer to Non Patent Literature 1). In addition, a treatment application aimed at treatment of nicotine dependence by smoking cessation is also known. Many treatment applications are installed and used in smartphones.

Typical smartphone applications can be freely downloaded from a predetermined download site (so-called app store), and can be installed and used in smartphones. In contrast, since the treatment application is a medical device (medical software), the treatment application cannot be freely used by anyone. To use the treatment application, an ID and a password, which are provided when a patient has been interviewed and diagnosed by a doctor and the doctor has determined that the treatment application is necessary and has prescribed the treatment application, are required.

In the related art, the ID and the password are issued by a medical institution. The medical institution inputs patient information from a terminal to issue a patient ID and a password, records the patient information and the patient ID in a two-dimensional code, and hands over the two-dimensional code and the password separately to the patient in a form of data or paper medium. However, in a situation in which the medical institution should prescribe a patient ID and a password of a treatment application to every patient, the burden on a medical professional becomes heavy.

In contrast, there is known a system in which a doctor who diagnosed a patient does not perform a computer operation for issuing a user ID and a password, and an ID and a password of a treatment application is automatically issued in response to a request from the treatment application installed in a user terminal (for example, refer to Patent Literature 1).

In the system described in Patent Literature 1, a patient who has installed the treatment application in the user terminal makes a code display request from a management screen displayed on a medical institution terminal installed in a lobby, an examination room, or the like in the medical institution after receiving a doctor's diagnosis, a management server received the request from the medical institution terminal issues the user ID and the password, generates a two-dimensional code image on which information on the user ID and the password is recorded, and causes the medical institution terminal to display the two-dimensional code image. When the user who has made the code display request reads out the two-dimensional code image displayed on the medical institution terminal by a function of the treatment application, a system registration request is transmitted from the treatment application to the management server, the treatment application which has made the request is registered as a normal application, and a functional restriction on the treatment application is released. Then, it is possible to use the treatment application without the functional restriction by log-in using the user ID and the password.

However, in the system described in Patent Literature 1, a special mechanism that gives a restriction on some functions of the treatment application until the user ID and the password are registered is required, and thus a treatment application that does not include the function restricting mechanism is not applicable.

In addition, there is also known a system in which the treatment application is made available for use by reading a predetermined activation code (two-dimensional code) with a smartphone (for example, refers to Patent Literatures 2 and 3) .

In the system described in Patent Literature 2, when a decision is made to use the medical application as a result of diagnosis, a doctor or a person who receives a doctor's instruction issues a prescription (including an activation code generated in a form of a barcode or a two-dimensional code) through a system such as an electronic medical record. A patient who receives the prescription downloads and installs the medical application on a patient side terminal, activates the medical application, reads the activation code by using a code reader function of the terminal, and makes the medical application available for use.

In the system described in Patent Literature 3, the server receives a request for the activation code for executing the treatment application from an information processing terminal of a patient who is a treatment target by the treatment application, generates the activation code based on initial information that is input in advance by a medical professional and relates to a patient's illness that is being treated, and transmits the activation code to the information processing terminal of the patient. The information processing terminal of the patient executes a treatment application in a state in which the initial information is applied by using the activation code received from the server.

However, in the system described in Patent Literature 2, it is necessary for the doctor or the person who receives the doctor's instruction to issue the activation code, in which various pieces of information are recorded, through the system such as the electronic medical record. In addition, in the system described in Patent Literature 3, it is necessary for the medical professional to input the initial information on the patient's illness that is being treated and register the initial information in a server in advance as the assumption for issuing the activation code for executing the treatment application. Therefore, even in any of the systems in Patent Literatures 2 and 3, the burden on the medical professional becomes heavy.

### Citation List

### Patent Literatures

PTL 1: JP6611112B
PTL 2: JP2022-80050A
PTL 3: JP2022-70086A

### Non Patent Literature

NPL 1: Website ""Yawn" improves insomnia and recovers 15 trillion yen of economic loss", searched on 2022.9.12, Internet URL <https://alldtx.com/product-best/mentalhealth-best-product-pi ckup-best/yawn-susmed/>

### Summary of Invention

### Technical Problem

The invention has been made to solve the problems, and an object thereof is to make a treatment application available for use without placing a heavy burden on a medical professional. Solution to Problem

To solve the problem, in the invention, a management server issues a medical institution ID for identifying a medical institution that prescribes a treatment application as an ID required for initiation of use of the treatment application installed in a patient terminal, and the treatment application acquires the medical institution ID issued by the management server and sets the medical institution ID in the treatment application as information for making the treatment application available for use.

### Advantageous Effects of Invention

As described above, according to the invention, the ID required for initiation of use of the treatment application is the medical institution ID that is determined in advance for every medical institution, and does not include information on a patient. Therefore, it is not necessary for a medical professional in a medical institution that diagnoses the patient and prescribes the treatment application to perform any input work for making the treatment application available for use, and the patient can perform setting for making the treatment application available for use by setting the medical institution ID issued by the management server in the treatment application. In this way, according to the invention, the treatment application can be made available for use without placing a heavy burden on the medical professional in the medical institution.

### Brief Description of Drawings

[FIG. 1] FIG. 1 is a view illustrating an overall configuration example of a treatment application management system according to an embodiment.
[FIG. 2] FIG. 2 is a block diagram illustrating a functional configuration example of the treatment application management system of this embodiment.
[FIG. 3] FIG. 3 is a block diagram illustrating a functional configuration example of the treatment application management system of this embodiment.
[FIG. 4] FIG. 4 is a flowchart illustrating an operation example of the treatment application management system according to this embodiment.
[FIG. 5] FIG. 5 is a flowchart illustrating an operation example of the treatment application management system according to this embodiment.

### Description of Embodiments

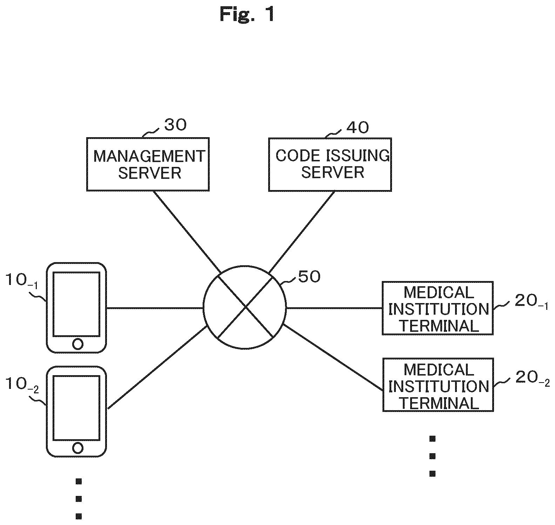

Hereinafter, an embodiment of the invention will be described based on the drawings. FIG. 1 is a view illustrating an overall configuration example of a treatment application management system according to this embodiment. As shown in FIG. 1, the treatment application management system of this embodiment includes a plurality of patient terminals 10₋₁, 10₋₂, ...(hereinafter, referred to as "patient terminals 10" unless particularly specified), and a plurality of medical institution terminals 20₋₁, 20₋₂, ... (hereinafter, referred to as "medical institution terminals 20" unless particularly specified), a management server 30, and a code issuing server 40. The patient terminals 10, the medical institution terminals 20, the management server 30, and the code issuing server 40 are connected to each other via a communication network 50 such as the Internet and a mobile phone network.

The plurality of patient terminals 10₋₁, 10₋₂, ... are terminals used by individual patients who install the treatment application and perform a treatment of an illness, and are constituted, for example, by a smartphone, a tablet, or a personal computer. The plurality of medical institution terminals 20₋₁, 20₋₂, ... are terminals used in individual medical institutions which diagnose a patient and prescribes the treatment application, and are constituted, for example, by a smartphone, a tablet, a personal computer, or a dedicated terminal for medical use.

The management server 30 and the code issuing server 40 perform a process relating to initiation of use of the treatment application that is installed and used in each of the patient terminals 10. Here, the management server 30 is, for example, a server that is operated by a business entity that provides the treatment application, and issues a medical institution ID required for initiation of use of the treatment application. After the use of the treatment application is initiated, the management server 30 also executes a process relating to a treatment by the treatment application in cooperation with the treatment application and each of the medical institution terminals 20.

In addition, the code issuing server 40 generates code information including the medical institution ID issued by the management server 30, and provides the code information to the medical institution terminal 20. The code issuing server 40 may be, for example, a server that is operated by the business entity that provides the treatment application, or a server that is operated by a different business entity. Note that, the management server 30 may include a medical institution ID issuing unit 31, and a code information generation unit 41 without separating the management server 30 and the code issuing server 40.

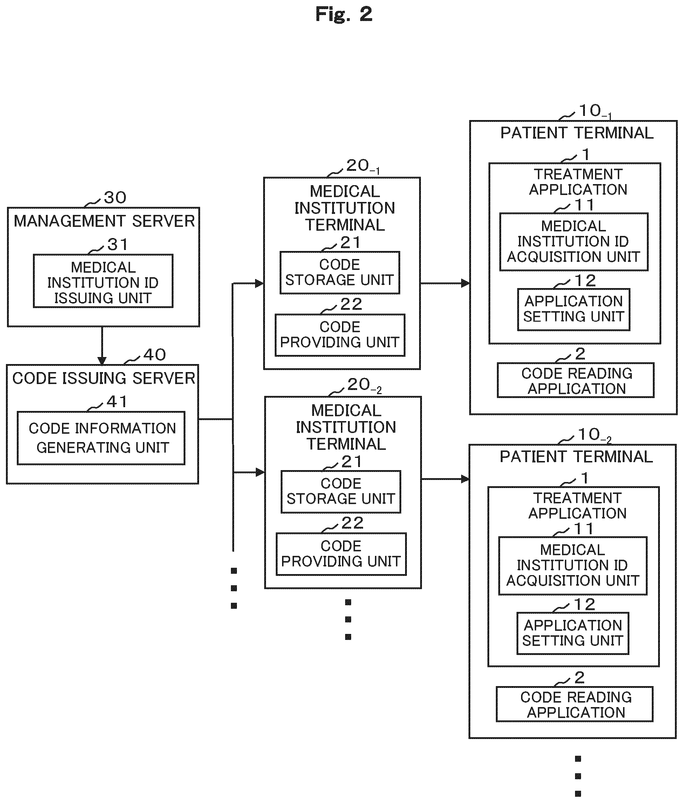

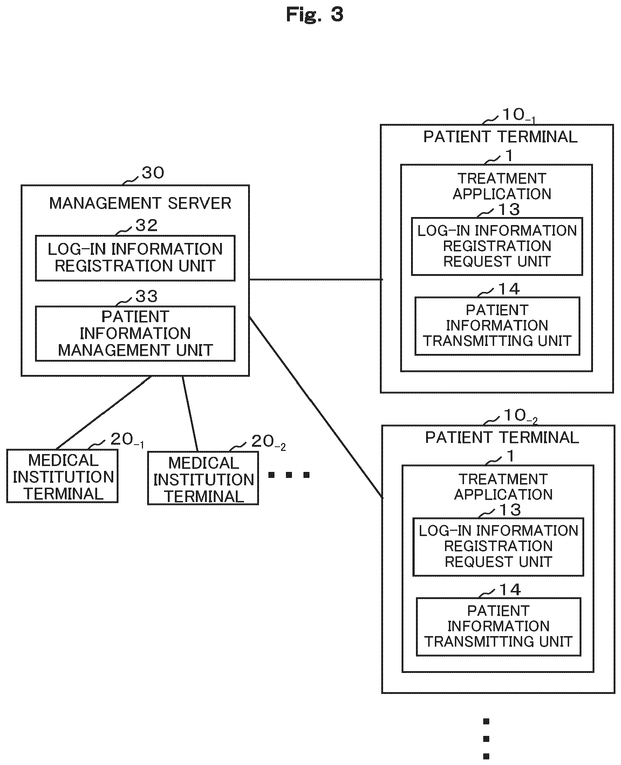

FIG. 2 and FIG. 3 are block diagrams illustrating a functional configuration example of the treatment application management system of this embodiment. FIG. 2 shows a functional configuration relating to a process until the medical institution ID is set in the treatment application, and FIG. 3 shows a functional configuration relating to a process after the medical institution ID is set in the treatment application.

As shown in FIG. 2, as the functional configuration relating to the process until setting the medical institution ID in a treatment application 1, the treatment application 1 of the patient terminal 10 includes a medical institution ID acquisition unit 11 and an application setting unit 12, the medical institution terminal 20 includes a code storage unit 21 and a code providing unit 22, the management server 30 includes the medical institution ID issuing unit 31, and the code issuing server 40 includes the code information generation unit 41. A code reading application 2 (corresponding to a code reading unit in the appended claims) is also stored in the patient terminal 10 in addition to the treatment application 1. Note that, the code reading unit may be provided in the treatment application 1.

In addition, as shown in FIG. 3, as the functional configuration relating to the process after setting the medical institution ID in the treatment application 1, the treatment application 1 includes a log-in information registration request unit 13 and a patient information transmitting unit 14, and the management server 30 includes a log-in information registration unit 32 and a patient information management unit 33.

Functional blocks shown in FIG. 2 and FIG. 3 may be configured by any of a hardware, digital signal processor (DSP), and software. For example, in a case of being configured by the software, the functional blocks actually include a CPU, a RAM, a ROM, and the like of a computer built in each of the patient terminal 10, the medical institution terminal 20, the management server 30, and the code issuing server 40, and are realized when a program stored in a storage medium such as the RAM, the ROM, a hard disk, or a semiconductor memory operates. Particularly, the functional configuration of the treatment application 1 is realized when a treatment application program installed in the patient terminal 10 operates.

In FIG. 2, the medical institution ID issuing unit 31 of the management server 30 issues the medical institution ID for identifying individual medical institutions, and provides the issued medical institution ID to the code issuing server 40. In this embodiment, the medical institution ID issuing unit 31 issues a plurality of medical institution IDs corresponding to a plurality of medical institutions regardless of whether or not the medical institution prescribes the treatment application 1 to a patient, and provides the plurality of medical institution IDs to the code issuing server 40.

Here, "regardless of whether or not the medical institution prescribes the treatment application 1 to a patient" means in advance before the medical institution prescribes the treatment application 1. For example, when information on a medical institution that has determined to introduce the treatment application 1 is registered in the management server 30 for the first time, the medical institution ID issuing unit 31 issues a medical institution ID for identifying the medical institution that is a registration destination and provides the medical institution ID to the code issuing server 40. The medical institution ID issuing unit 31 issues the medical institution ID whenever the information on the medical institution is registered in the management server 30.

The code information generation unit 41 of the code issuing server 40 generates code information in which the medical institution ID is recorded, and provides the generated code information to the medical institution terminal 20 of a medical institution corresponding to the medical institution ID. The code information does not include both log-in information and patient initial information to be described later. For example, the code information is a two-dimensional code such as a QR code (registered trademark). Alternatively, the code information may be a barcode, or other computer-readable codes.

In this embodiment, the code information generation unit 41 generates a plurality of pieces of code information in which corresponding medical institution IDs are recorded for a plurality of medical institutions regardless of whether or not the medical institutions prescribe the treatment application 1 to patients, and provides the plurality of pieces of code information to the plurality of medical institution terminals 20. For example, as described above, whenever information on the medical institution is registered in the management server 30, and the medical institution ID of the medical institution is provided from the management server 30 to the code issuing server 40, the code information generation unit 41 generates code information including the provided medical institution ID, and provides the generated code information to a corresponding medical institution terminal 20.

A method of providing the code information is arbitrary. For example, the code information generating unit 41 can transmit the code information to the medical institution terminal 20 via the communication network 50. The code information generating unit 41 may record the code information in a removable storage medium, and an administrator may deliver the removable storage medium to the medical institution terminal 20. Alternatively, the code information generating unit 41 may provide the code information to the medical institution terminal 20 via a data transfer website managed by the management server 30 or the code issuing server 40.

The code storage unit 21 of the medical institution terminal 20 acquires and stores the code information provided from the code issuing server 40. The code storage unit 21 stores the code information in which the medical institution ID of the medical institution is recorded regardless of whether or not the medical institution prescribes the treatment application 1 to a patient (in advance before the medical institution prescribes the treatment application 1 to the patient).

The code providing unit 22 provides the code information stored in the code storage unit 21 to the patient terminal 10. The code information is provided to the patient terminal 10 after the medical institution prescribes the treatment application 1 to the patient. For example, when the medical professional instructs provision of the code information by operating the medical institution terminal 20, the code providing unit 22 receives the instruction, reads out the code information from the code storage unit 21, and provides the code information to the patient terminal 10.

A method of providing the code information is arbitrary. For example, the code providing unit 22 may cause a display device of the medical institution terminal 20 to display the code information, and the code information may be provided by reading the code information with the code reading application 2 of the patient terminal 10. Instead of causing the display device of the medical institution terminal 20 to display the code information, the code providing unit 22 may print the code information on a paper medium by using a printing device connected to the medical institution terminal 20. In this case, the code information recorded on the paper medium is read with the code reading application 2 of the patient terminal 10, and the code information is provided. The code providing unit 22 may record the code information on a removable storage medium, and the patient terminal 10 may read the code information from the removable storage medium, and the code information may be provided. Alternatively, the code providing unit 22 may provide the code information to the patient terminal 10 via a data transfer website managed by the management server 30.

The medical institution ID acquisition unit 11 of the treatment application 1 acquires the medical institution ID issued by the medical institution ID issuing unit 31 of the management server 30. For example, in a case where the code information is provided to the patient terminal 10 from the medical institution terminal 20 by reading of the code information with the code reading application 2, the medical institution ID acquisition unit 11 acquires the medical institution ID obtained by reading and decrypting the code information with the code reading application 2 from the code reading application 2. In a case where the code information is provided to the patient terminal 10 from the medical institution terminal 20 via the removable storage medium or the data transfer website, the medical institution ID acquisition unit 11 acquires the medical institution ID by decrypting the provided code information.

The application setting unit 12 sets the medical institution ID acquired by the medical institution ID acquisition unit 11 in the treatment application 1 as information for making the treatment application 1 available for use. Description of "making the treatment application 1 available for use" means a state in which the treatment application 1 can perform a process relating to a treatment in cooperation with the medical institution terminal 20 via the management server 30.

That is, after a patient initiates the use of the treatment application 1, various pieces of information on the treatment (information indicating a current symptom or an execution situation of the treatment application 1, and the like) are registered in the management server 30 from the treatment application 1, and the information can be viewed from the medical institution terminal 20, but it is necessary that the information can be viewed only from the medical institution terminal 20 linked to the treatment application 1. The medical institution ID is used for link between the treatment application 1 and the medical institution terminal 20. Accordingly, before the medical institution ID is set in the treatment application 1, the treatment application 1 is in an unusable state, and the medical institution ID is information that needs to be set in the treatment application 1 when initiating use of the treatment application 1.

In FIG. 3, the log-in information registration request unit 13 of the treatment application 1 makes a log-in information registration request, which is executable due to setting of the medical institution ID by the application setting unit 12, to the management server 30. For example, the log-in information registration request unit 13 causes a display device of the patient terminal 10 to display a log-in information registration screen, for example, with completion of setting of the medical institution ID set as a trigger, and promotes a patient to register the log-in information. Note that, the log-in information registration request may be made by the patient by manually displaying the registration screen at an arbitrary timing after completion of the setting of the medical institution ID.

In this embodiment, when the patient actually uses the treatment application 1, the patient is required to log in the treatment application 1. A user ID and a password of the patient are used for the log-in. For example, an email address of the patient terminal 10 can be used as the user ID. When the patient inputs the user ID and the password by operating the registration screen, the log-in information registration request unit 13 transmits the input user ID and password, and the medical institution ID acquired by the medical institution ID acquisition unit 11 to the management server 30 to make the log-in information registration request.

The log-in information registration unit 32 of the management server 30 registers the log-in information that is used by the patient when logging in the treatment application 1 in the management server 30 in response to the log-in information registration request transmitted from the treatment application 1. In the registration, the log-in information registration unit 32 registers the user ID in association with the medical institution ID transmitted from the treatment application 1 together with the log-in information. As described above, in this embodiment, when the medial institution ID is not set in the treatment application 1, the log-in information cannot be registered in the management server 30.

After the log-in information is registered in the management server 30 in response to the log-in information registration request made by the log-in information registration request unit 13, the patient information transmitting unit 14 of the treatment application 1 receives an input of patient initial information to be set when initiating the treatment using the treatment application 1, and transmits the input patient initial information to the management server 30.

For example, the patient information transmitting unit 14 displays a patient initial information input screen on the display device of the patient terminal 10, for example, with reception of notification of log-in information registration completion from the management server 30 set as a trigger, and promotes the patient to input the patient initial information. The patient initial information includes, for example, patient card number, a name, a sex, a date of birth, another illness that is being treated (concomitant illness), and information (current symptom and the like) relating to the illness that is being treated with the treatment application 1. When the patient inputs the patient initial information by operating the input screen, the patient information transmitting unit 14 transmits the input patient initial information to the management server 30. Note that, the input of the patient initial information may be performed by the patient by manually displaying the input screen at an arbitrary timing after the registration of the log-in information is completed.

The patient information management unit 33 stores the patient initial information transmitted from the treatment application 1 in a storage medium, and manages the patient initial information in a state viewable from the medical institution terminal 20 linked to the treatment application 1 with the medical institution ID. That is, when the medical institution terminal 20 accesses the management server 30 by using the medical institution ID, the patient information management unit 33 provides the patient initial information registered in association with the user ID linked to the medical institution ID to the medical institution terminal 20 in a viewable state.

The patient information management unit 33 provides not only the patient initial information but also various pieces of information related to the treatment, which are sequentially registered in the management server 30 from the treatment application 1 after initiating the use of the treatment application 1, to the medical institution terminal 20. A medical professional can grasp the patient's illness or a status of the treatment (numerical values vary in which manner, whether the use of the treatment application 1 is continued, or the like) by viewing the information on the treatment, and can contact the patient as necessary. Note that, the patient information management unit 33 does not provide the user ID (email address) and the password to the medical institution terminal 20.

The patient information management unit 33 may manage the patient initial information transmitted from the treatment application 1 in a state editable from the medical institution terminal 20 linked to the treatment application 1 with the medical institution ID. For example, in a case where the medical professional who has viewed the patient initial information registered in the management server 30 compares the input information with memory of the medical professional and medical record information in diagnosis at the time of prescribing the treatment application 1, and recognizes that the input is incorrect, or the like, the medical professional may be allowed to correct the information. In addition, in a case where the medical professional compares the information with the memory of the medical professional and the medical record information in diagnosis, or input information on another patient, and recognizes that an input omission is present, or the like, the medical professional may be allowed to input additional information.

The patient information management unit 33 may manage not only the patient initial information but also various pieces of information on the treatment, which are sequentially registered in the management server 30 from the treatment application 1 after initiation of use of the treatment application 1, in a state editable from the medical institution terminal 20 linked to the treatment application 1 with the medical institution ID. For example, in a case where the information that is transmitted from the treatment application 1 to the management server 30 and is registered therein is compared with the medical record information that is used daily in the medical care by the medical professional, and it is recognized that an input error or an input omission exists, or the like, the medical professional may be allowed to edit the information.

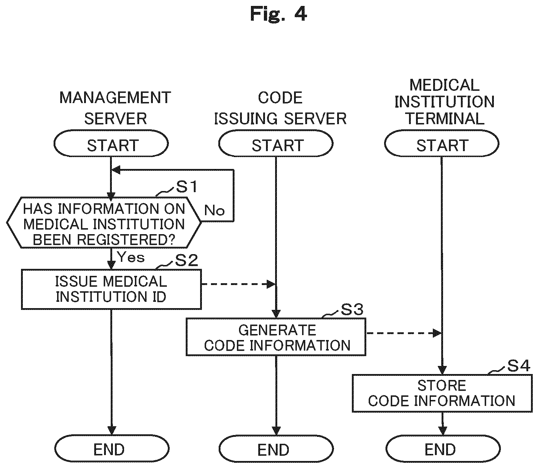

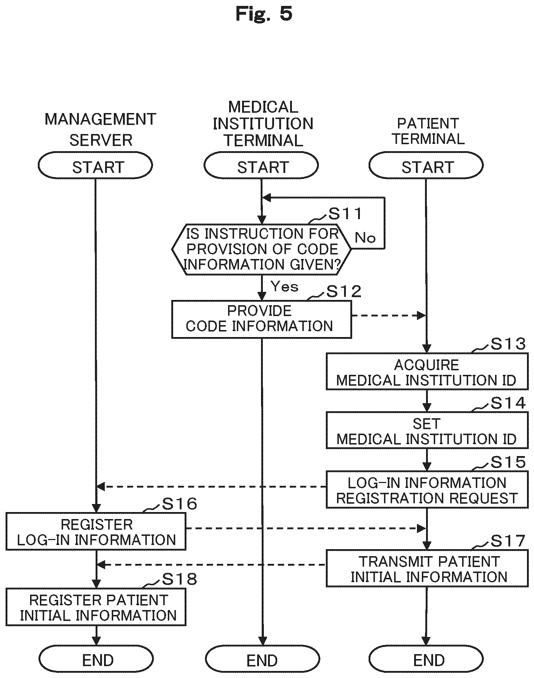

FIG. 4 and FIG. 5 are flowcharts illustrating an operation example of the treatment application management system according to this embodiment configured as described above. FIG. 4 shows a process until the code information is stored in the medical institution terminal 20, and FIG. 5 shows a process after a medical institution prescribes the treatment application 1 to a patient.

In FIG. 4, first, the medical institution ID issuing unit 31 of the management server 30 determines whether or not information on a medical institution that has determined to introduce the treatment application 1 is registered in the management server 30 (step S1). Here, the determination in step S1 is repeated at predetermined time intervals until the information on the medical institution is registered in the management server 30. When the information on the medical institution is registered in the management server 30, the medical institution ID issuing unit 31 issues a medical institution ID for identifying the medical institution, and provides the issued medical institution ID to the code issuing server 40 (step S2).

The code information generation unit 41 of the code issuing server 40 generates code information in which the medical institution ID provided from the management server 30 is recorded, and provides the generated code information to the medical institution terminal 20 of the medical institution corresponding to the medical institution ID (step S3). The code storage unit 21 of the medical institution terminal 20 acquires and stores the code information provided from the code issuing server 40 (step S4). As described above, the process of the flowchart shown in FIG. 4 is completed.

In FIG. 5, first, the code providing unit 22 of the medical institution terminal 20 determines whether or not a medical professional who has prescribed the treatment application 1 performs an operation of giving an instruction for provision of the code information on the medical institution terminal 20 (step S11). In a case where the instruction has been given, the code providing unit 22 provides the code information stored in the code storage unit 21 to the patient terminal 10 of a patient to whom the treatment application 1 has been prescribed (step S12).

In the patient terminal 10, the medical institution ID acquisition unit 11 of the treatment application 1 acquires the medical institution ID, which is obtained by reading and decrypting the code information by the code reading application 2, from the code reading application 2 (step S13). Then, the application setting unit 12 sets the medical institution ID acquired by the medical institution ID acquisition unit 11 in the treatment application 1 as information for making the treatment application 1 available for use (step S14).

Next, the log-in information registration request unit 13 displays a log-in information registration screen on the patient terminal 10, and transmits a user ID and a password which are input through the registration screen, and the medical institution ID acquired by the medical institution ID acquisition unit 11 to the management server 30 to make a log-in information registration request (step S15). In response to the log-in information registration request transmitted from the treatment application 1, the log-in information registration unit 32 of the management server 30 registers the log-in information in the management server 30 in association with the medical institution ID (step S16).

Then, the patient information transmitting unit 14 of the treatment application 1 displays a patient initial information input screen on the patient terminal 10 with reception of a notification of log-in information registration completion from the management server 30 set as a trigger, and transmits the patient initial information input through the input screen to the management server 30 (step S17). The patient information management unit 33 of the management server 30 stores the patient initial information transmitted from the treatment application 1 in a storage medium, and manages the patient initial information in a state viewable and editable from the medical institution terminal 20 linked to the treatment application 1 with the medical institution ID (step S18). As described above, the process in the flowchart shown in FIG. 5 is completed.

As described above in detail, in this embodiment, the medical institution ID (ID for identifying a medical institution that has prescribed a treatment application) required for initiation of use of the treatment application 1 installed in the patient terminal 10 is issued in the management server 30, and the medical institution ID is acquired and set in the treatment application 1, thereby making the treatment application 1 available for use.

In addition, in this embodiment, after the medical institution ID is set in the treatment application 1, the treatment application 1 makes a log-in information registration request to the management server 30 to register the log-in information. In addition, in this embodiment, after the log-in information is registered, the patient initial information is transmitted from the treatment application 1 to the management server 30 to be registered.

As described above, in this embodiment, the information that is used to make the treatment application 1 available for use is the medical institution ID that is determined in advance for every medical institution, and does not include log-in information and information on a patient. Therefore, it is not necessary for a medical professional in the medical institution to perform any input work for issuing an ID, and the medical institution ID can be issued in the management server 30. In addition, the medical institution ID can also be provided to the medical institution terminal 20 in advance before the medical professional diagnoses the patient and prescribes the treatment application 1. In addition, after the treatment application 1 is prescribed, the patient can perform setting for making the treatment application 1 available for use by setting the medical institution ID provided from the medical institution in the treatment application 1. In addition, after setting the medical institution ID in the treatment application 1, the patient inputs the log-in information and the patient initial information from the treatment application 1 to be registered in the management server 30. Therefore, it is also not necessary for the medical professional in the medical institution to perform a work for registering the log-in information and the patient initial information for making the treatment application 1 actually available for use. In this way, according to this embodiment, a patient can make the treatment application 1 available for use without placing a heavy burden on the medical professional in the medical institution.

Note that, in the above-described embodiment, description has been given of a configuration in which the management server 30 is a server that is operated by a business entity who provides the treatment application 1, and the management server 30 is provided with the functional configurations 31 to 33, but the invention is not limited thereto. For example, a configuration may be employed that includes a first management server including the medical institution ID issuing unit 31 to issue the medical institution ID, and a second management server including the log-in information registration unit 32 and the patient information management unit 33 and executing a process relating to a treatment using the treatment application 1, such that the second management server may be operated by a business entity providing the treatment application 1, and the first management server may be operated by another business entity. In addition, the first management server may include the code information generation unit 41 in addition to the medical institution ID issuing unit 31. In this case, the first management server may be a plurality of servers operated by individual medical institutions. Even if the configuration in which the medical institution ID is issued by the management servers operated by the medical institutions, it is also not necessary for medical professionals in the medical institutions to perform any input work for issuing the ID.

In addition, in the above-described embodiment, description has been given of an example in which the code issuing server 40 is provided, and the code issuing server 40 encodes the medical institution ID and provides the medical institution ID to the medical institution terminal 20, but the invention is not limited thereto. That is, the medical institution ID issued by the medical institution ID issuing unit 31 may be provided to the medical institution terminal 20, and the medical institution ID may be provided from the medical institution terminal 20 to the treatment application 1 of the patient terminal 10.

In addition, in the above-described embodiment, description has been given of a configuration in which log-in to the treatment application 1 is performed by using log-in information registered in the management server 30, but log-in to the treatment application 1 may be unnecessary. In this case, for example, it is considered that an application ID transmitting unit is provided instead of the log-in information registration request unit 13, and an application ID for identifying the prescribed treatment application 1, and the medical institution ID are registered in the management server 30 in association with each other instead of a configuration in which the user ID and the medical institution ID are registered in the management server 30 in association with each other. In addition, in this case, after the treatment application 1 enters an available state due to setting of the medical institution ID by the application setting unit 12, the patient information transmitting unit 14 receives an input of patient initial information to be set when initiating a treatment using the treatment application 1, and transmits the input patient initial information to the management server 30.

In addition, in the above-described embodiment, description has been given of an example in which the medical institution ID is issued and code information is stored in the medical institution terminal 20 regardless of whether or not a medical institution prescribes the treatment application 1 to a patient (in advance before the medical institution prescribes the treatment application 1 to the patient), but the invention is not limited thereto. For example, when the medical institution prescribes the treatment application 1 to the patient, an ID issuing request may be transmitted from the medical institution terminal 20 to the management server 30 to issue the medical institution ID.

In addition, the above-described embodiment is merely an example of an embodiment for carrying out the invention, and the technical scope of the invention should not be interpreted as being limited by the embodiment. That is, the invention can be carried out in various forms without departing from the gist and characteristics of the invention.

### Reference Signs List

1: treatment application
10: patient terminal
11: medical institution ID acquisition unit
12: application setting unit
13: log-in information registration request unit
14: patient information transmitting unit
20: medical institution terminal
21: code storage unit
22: code providing unit
30: management server
31: medical institution ID issuing unit
32: log-in information registration unit
33: patient information management unit
40: code issuing server
41: code information generation unit

## Claims

1. A treatment application management system configured to perform a process relating to initiation of use of a treatment application that is installed and used in a patient terminal, comprising:
the treatment application installed in the patient terminal; and
a management server configured to issue an ID required for the initiation of use of the treatment application,
wherein the management server includes a medical institution ID issuing unit configured to issue a medical institution ID for identifying a medical institution that prescribes the treatment application, and
the treatment application includes,
a medical institution ID acquisition unit configured to acquire the medical institution ID issued by the medical institution ID issuing unit, and
an application setting unit configured to set the medical institution ID acquired by the medical institution ID acquisition unit as information for making the treatment application available for use.

2. The treatment application management system according to claim 1,
wherein the treatment application further includes a log-in information registration request unit configured to make a log-in information registration request, which is executable due to setting of the medical institution ID by the application setting unit, to the management server, and
the management server further includes a log-in information registration unit configured to register log-in information that is used when a patient who uses the treatment application logs in the treatment application in response to the log-in information registration request transmitted from the treatment application.

3. The treatment application management system according to claim 2,
wherein the management server includes,
a first management server including the medical institution ID issuing unit, and
a second management server including the log-in information registration unit.

4. The treatment application management system according to claim 1,
wherein the treatment application further includes a patient information transmitting unit configured to receive an input of patient initial information to be set when initiating a treatment using the treatment application after the medical institution ID is set by the application setting unit, and to transmit the input patient initial information to the management server, and
the management server further includes a patient information management unit configured to manage the patient initial information transmitted from the treatment application in a state viewable from a medical institution terminal that is used in the medical institution.

5. The treatment application management system according to claim 2,
wherein the treatment application further includes a patient information transmitting unit configured to receive an input of patient initial information to be set when initiating a treatment using the treatment application after the log-in information is registered by the log-in information registration unit, and to transmit the input patient initial information to the management server, and
the management server further includes a patient information management unit configured to manage the patient initial information transmitted from the treatment application in a state viewable from a medical institution terminal that is used in the medical institution.

6. The treatment application management system according to claim 4 or 5,
wherein the patient information management unit manages the patient initial information transmitted from the treatment application in a state editable from the medical institution terminal.

7. The treatment application management system according to claim 2,
wherein the treatment application further includes a patient information transmitting unit configured to receive an input of patient initial information to be set when initiating a treatment using the treatment application after the log-in information is registered by the log-in information registration unit, and to transmit the input patient initial information to the management server,
the management server further includes a patient information management unit configured to manage the patient initial information transmitted from the treatment application in a state viewable from a medical institution terminal that is used in the medical institution,
the management server or a code issuing server that is provided with the medical institution ID from the management server includes a code information generation unit configured to generate code information that does not include both the log-in information and the patient initial information, and includes the medical institution ID, and
the medical institution ID acquisition unit of the patient terminal acquires the medical institution ID obtained by reading and decrypting the code information by a code reading unit of the patient terminal.

8. The treatment application management system according to any one of claims 1 to 5,
wherein the management server or a code issuing server that is provided with the medical institution ID from the management server includes a code information generation unit configured to generate code information including the medical institution ID and provides the generated code information to a medical institution terminal that is used in the medical institution, and
the medical institution ID acquisition unit of the patient terminal acquires the medical institution ID obtained by reading and decrypting the code information displayed on a display device of the medical institution terminal by a code reading unit of the patient terminal.

9. The treatment application management system according to any one of claims 1 to 5,
wherein the medical institution ID issuing unit issues a plurality of medical institution IDs corresponding to a plurality of medical institutions regardless of whether or not the medical institutions prescribe the treatment application.

10. The treatment application management system according to claim 8,
wherein the medical institution ID issuing unit issues a plurality of medical institution IDs corresponding to a plurality of medical institutions regardless of whether or not the medical institution prescribes the treatment application, and
the code information generation unit generates a plurality of pieces of code information in which the corresponding medical institution IDs are recorded for the plurality of medical institutions regardless of whether or not the medical institutions prescribe the treatment application.

11. A treatment application program that is a program of a treatment application installed and used in a patient terminal, the program causing a computer to function as:
a medical institution ID acquisition unit acquiring a medical institution ID that is an ID issued from a management server as an ID required for initiation of use of the treatment application and identifies a medical institution that prescribes the treatment application; and
an application setting unit setting the medical institution ID acquired by the medical institution ID acquisition unit as information for making the treatment application available for use.

12. The treatment application program according to claim 11, the program further causing the computer to function as:
a log-in information registration request unit making a log-in information registration request, which is executable due to setting of the medical institution ID by the application setting unit, to the management server.

13. The treatment application program according to claim 12, the program further causing the computer to function as:
a patient information transmitting unit receiving an input of patient initial information to be set when initiating a treatment using the treatment application after the log-in information is registered in the management server in response to the log-in information registration request made by the log-in information registration request unit, and to transmit the input patient initial information to the management server.
